# EUROPEAN PATENT APPLICATION

(11) **EP 4 278 970 A2**
(43) Date of publication of application: **22.11.2023**
(21) Application number: 23201807.7
(22) Date of filing: 04.06.2007
(51) Int. Cl.: A61B 5/1455

(54) **PARAMETER UPGRADE SYSTEM**

(30) Priority: 05.06.2006 US 811001 P
(62) Divisional of application: 19205949.1
(71) Applicant: Masimo Corporation, Irvine, CA 92618 (US)
(72) Inventor: The designation of the inventor has not yet been filed
(74) Representative: Vossius & Partner Patentanwälte Rechtsanwälte mbB

(57) **Abstract**

The present invention discloses a parameter upgrade system comprising: a physiological monitor having a sensor port configured to attach and communicate with a sensor; a processor board in communications with the sensor port having a board digital signal processor (DSP); a plurality of firmware instructions residing on the processor board and executable by the board DSP so as to calculate a plurality of physiological parameters in response to a sensor signal received from the sensor and determine an enable state for each of the plurality of physiological parameters; and a plurality of handheld upgrade tools individually attachable to the sensor port in lieu of the sensor, wherein each of the plurality of handheld upgrade tools comprises: a tool digital signal processor (DSP) configured to provide changes to the plurality of firmware instructions and configured to, update, based on a verification that a parameter upgrade is available, an enable state of a physiological parameter to make the physiological parameter available for output by the monitor, wherein making the physiological parameter available for output by the monitor enables the sensor port to calculate at least one additional physiological parameter in response to the sensor; a sensor port connector that mates directly with the sensor port and provides a communications path between the tool DSP and the board DSP; an I/O port connector that provides communications between the tool DSP and an external digital device; and an information element that identifies the handheld upgrade tool to the board DSP.

## Description

### CROSS-REFERENCE TO RELATED APPLICATIONS

The present application claims priority benefit under 35 U.S.C. § 119(e) to U.S. Provisional Patent Application Serial No. 60/811,001, filed June 5, 2006, entitled *"Parameter Upgrade System,"* incorporated herein by reference.

### BACKGROUND OF THE INVENTION

Physiological monitoring systems include patient monitors and corresponding noninvasive sensors for measuring constituents of circulating blood. Such patient monitoring systems have gained rapid acceptance in a wide variety of medical applications, including surgical wards, intensive care and neonatal units, general wards, home care, physical training and virtually all types of monitoring scenarios. A noninvasive sensor having light emitting diodes (LEDs) transmits optical radiation into a tissue site. A detector responds to the intensity of the optical radiation after absorption by pulsatile blood flow within the tissue site. Based upon this response, a patient monitor determines measurements for physiological parameters such as oxygen saturation, pulse rate and perfusion among others. Advanced patient monitors utilizing multiple wavelength sensors determine measurements for other physiological parameters, such as carboxyhemoglobin and methemoglobin, as examples.

### SUMMARY OF THE INVENTION

A parameter upgrade system works in conjunction with a physiological monitoring system to advantageously allow a manufacturer to stock and distribute processor boards capable of measuring various combinations of physiological parameters without assigning a multitude of part numbers for each of these possible combinations. Also a parameter upgrade system easily configures processor board firmware according to the desired parameters. Firmware configuration of a processor board can be made at a place of board production, at a place of board integration into a host instrument and at end-user facilities, such as clinics or hospitals.

A parameter upgrade system advantageously uses a relatively small update tool that plugs into the sensor port of a physiological monitor so as to custom-configure the monitor's processor board with added physiological parameters. Each parameter can be added individually by a specific update tool. Additional parameters can be added in future upgrades as user requirements change. Upgrade tools can interface with various computer platforms, referred to herein generically as PCs, and be flexibly programmed, uploaded and downloaded utilizing PC-based manufacturer and field applications. Accordingly, upgrade tools have the ability to bring processor board firmware up to date and to capture and upload the history and status of multiple processor boards.

As used herein, "processor boards" refers to the hardware, including electrical and electronic components and circuits; and firmware or software, or various combinations of firmware and software, including algorithms, programs, processes, procedures and data stored in non-volatile memory or otherwise, for interfacing to a physiological monitoring system, communicating with an attached sensor or sensors and/or computing, calculating or otherwise deriving physiological parameter measurements, among other functions. Although processor board hardware and firmware are typically implemented on a printed circuit board (PCB), one of ordinary skill in the art will recognize that such functions can be implemented in various forms on various substrates including flexible circuits, hybrid circuits and ceramic substrates, to name a few.

In an embodiment, a parameter upgrade system functions in conjunction with physiological monitoring systems that include low noise optical sensors and pulse oximetry monitors, such as any of LNOP^{®} adhesive or reusable sensors, SofTouch^{™} sensors, Hi-Fi Trauma^{™} or Blue^{™} sensors; and any of Radical^{®}, SatShare^{™}, Rad-9^{™}, Rad-5^{™}, Rad-5v^{™} or PPO+^{™} Masimo SET^{®} pulse oximeters, all available from Masimo Corporation ("Masimo"), Irvine, CA. Physiological monitoring systems also include multiple wavelength sensors and corresponding noninvasive blood parameter monitors, such as Rainbow^{™} adhesive and reusable sensors and RAD-57^{™} and Radical-7^{™} monitors for measuring SpO₂, pulse rate, perfusion index, signal quality, HbCO and HbMet among other parameters. The Rainbow^{™} sensors and RAD-57^{™} and Radical-7^{™} monitors are available from Masimo Corporation, Irvine, CA.

In other embodiments, low noise sensors are as described in at least U.S. Pat. Nos. 5,782,757. Patient monitors capable of reading through motion-induced noise are as described in at least U.S. Pat. Nos. 6,770,028; 6,658,276; 6,157,850; 6,002,952; 5,769,785; 5,758,644 and 5,632,272, all incorporated by reference herein. Further, noninvasive sensors include multiple wavelength optical sensors, such as described in U.S. Pat. App. No. 11/376,013, filed March 1, 2006, entitled *Multiple Wavelength Sensor Emitters*; and physiological monitors include noninvasive blood parameter monitors, such as described in U.S. Pat. App. No. 11/367,033, filed March 1, 2006, entitled Noninvasive Multi-Parameter Patient Monitor, both patent applications assigned to Masimo Laboratories, Inc., Irvine, CA and both incorporated by reference herein.
The following items are preferred embodiments of the invention.
1. A parameter upgrade system comprising:
   a physiological monitor having a sensor port configured to attach and communicate with a sensor;
   a processor board in communications with the sensor port having a board digital signal processor (DSP);
   a plurality of firmware instructions residing on the processor board and executable by the board DSP so as to calculate a plurality of physiological parameters in response to a sensor signal received from the sensor; and
   an plurality of upgrade tools individually attachable to the sensor port in lieu of the sensor so as to designate to the processor board which of the physiological parameters, if any, to calculate when the sensor is attached to the sensor port.
2. The parameter upgrade system according to item 1 wherein the upgrade tools each comprise:
   a tool digital signal processor (DSP);
   a sensor port connector that mates with the sensor port and provides a communications path between the tool DSP and the board DSP;
   an I/O port connector that provides communications between the tool DSP and an external digital device; and
   an information element that identifies each of the upgrades tools to the board DSP.
3. The parameter upgrade system according to item 2 wherein at least one of the upgrade tools comprise a factory upgrade tool having the tool DSP programmed to communicate to the board DSP to make a specific physiological parameter available to the physiological monitor.
4. The parameter upgrade system according to item 3 wherein at least one of the upgrade tools comprise a board enable tool having the tool DSP programmed to enable the board DSP to calculate available physiological parameters to be displayed by physiological monitor.
5. The parameter upgrade system according to item 4 wherein at least one of the upgrade tools comprise an end-user tool having the tool DSP programmed to communicate to the board DSP to add available physiological parameters after the board DSP has been enabled.
6. The parameter upgrade system according to item 5 wherein at least one of the upgrade tools comprise a demo tool to indicate to the board DSP to verify available parameters.
7. The parameter upgrade system according to item 6 further comprising a manufacturer application executing on the PC and in communications with the tool DSP via the I/O port connector, wherein the manufacturer application programs the tool DSP with at least one of a tool parameter and a number of parameter updates that can be made with the tool.
8. The parameter upgrade system according to item 7 further comprising a field application executing on a PC and in communications with the tool DSP via the I/O port connector, wherein the field application displays on the PC at least one of the tool parameter and the number of parameter updates that can be made with the tool.
9. A parameter upgrade method comprising:
   providing a physiological monitor having a sensor port configured to communicate with a sensor so as to calculate a plurality of parameters associated with a physiological state of a living being;
   configuring the sensor port so as to communicate with any of a plurality of upgrade tools in lieu of the sensor; and
   attaching a first upgrade tool to the sensor port so as to communicate to the monitor which of the parameters should be made available for output by the monitor.
10. The parameter upgrade method according to item 9 further comprising attaching a demo tool to the sensor port so as to communicate to the monitor to verify available parameters.
11. The parameter upgrade method according to item 10 further comprising attaching an enable tool to the sensor port so as to communicate to the monitor to enable output measurement data for the available parameters.
12. The parameter upgrade method according to item 11 further comprising attaching a second upgrade tool to the sensor port so as to communicate to the monitor additional ones of the parameters that should be made available for output by the monitor.
13. The parameter upgrade method according to item 12 further comprising disabling the use of the first upgrade tool with the monitor after the enable tool has been attached to the monitor.
14. The parameter upgrade method according to item 13 further comprising configuring an I/O ports on at least one of upgrade tools to provide communications between the at least one upgrade tool and an external digital device.
15. The parameter upgrade method according to item 14 further comprising:
   interfacing a PC to the I/O port; and
   executing a manufacturing application on the PC so as to program the at least one upgrade tool with one of the parameters that can be made available to the monitor.
16. The parameter upgrade method according to item 14 further comprising executing a field application on the PC so as to read from the at least one upgrade tool the one of the parameters that an be made available to the monitor.
17. A parameter upgrade method comprising:
   providing a processor board having a sensor port that is adapted to communicate with a physiological sensor, the processor board capable of calculating a plurality of physiological parameters in response to a sensor signal received from the sensor;
   making a first set of the parameters available to the processor board via a first upgrading set of sensor port communications so that the processor board is configured to calculate the first set of parameters in response to the sensor signal; and
   enabling the available first set of parameters via an enable sensor port communications so that the processor board will calculate and output the available first set of parameters in response to the sensor signal.
18. The parameter upgrade method according to item 17 further comprising making available a second set of the parameters to the processor board via a second upgrading sensor port communications so that the processor board will calculate and output the second set of the parameters in response to the sensor signal.
19. The parameter upgrade method according to item 18 further comprising verifying the available first set of parameters by a processor board simulation that generates simulated outputs of available parameters.
20. The parameter upgrade method according to item 19 wherein the making a first set of the parameter available comprises:
   programming a first tool with a specific parameter of the first set of parameters and an upgrade count;
   connecting the first tool to the sensor port so as to communicate to the processor board to indicate that the specific parameter is available; and
   decrementing the upgrade count.
21. The parameter upgrade method according to item 20 wherein the enabling comprises connecting a second tool to the sensor port that is recognized by the processor board as authorizing the output of the available parameters.
22. The parameter upgrade method according to item 21 wherein the verifying comprises connecting a third tool to the sensor port that triggers the simulation.

### BRIEF DESCRIPTION OF THE DRAWINGS

**FIG. 1** is a general flow diagram of a parameter upgrade system;
**FIG. 2** is an illustration of an upgrade tool incorporated with a physiological monitoring system;
**FIG. 3** is a detailed block diagram of an upgrade tool incorporated with a physiological monitoring system;
**FIG. 4** is a flow diagram of a parameter upgrade process;
FIGS. 5A-D are top, perspective, front and side views of an upgrade tool;
**FIG. 6** is an exploded view of an upgrade tool;
FIGS. 7A-C are perspective, front and bottom partial assembly views of an upgrade tool;
FIGS. 8A-B is a flowchart of upgrade tool operational functions;
**FIGS. 9** is a flowchart of upgrade tool read functions;
**FIG. 10** is a flowchart of upgrade tool maintenance functions;
**FIG. 11** is an illustration of a field application graphical user interface (GUI);
**FIG. 12** is an illustration of a manufacturer application GUI;
**FIG. 13** is a block diagram of a network configuration for an upgrade tool;
**FIG. 14** is a block diagram of a wireless configuration for upgrade tools; and
**FIG. 15** is a block diagram of a two-tier parameter pricing structure.

### DETAILED DESCRIPTION OF THE PREFERRED EMBODIMENTS

### General Description

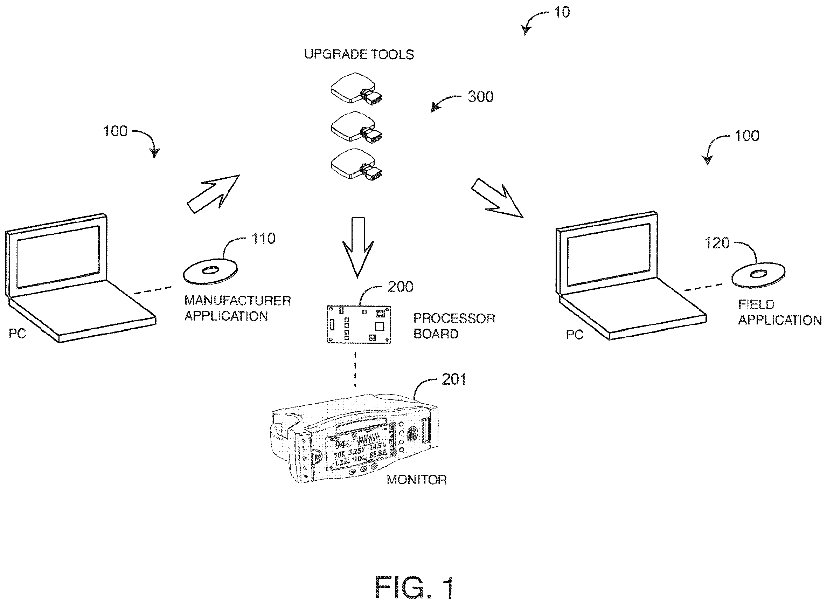

**FIG. 1** illustrates a parameter upgrade system **10** embodiment having application programs **100** and upgrade tools **300.** The parameter upgrade system **10** as a whole controls, facilitates, tracks and documents parameter additions and firmware version upgrades for physiological monitors **201** and, in particular, for a processor board **200** located within the monitor **201.** The parameter upgrade system **10** is used throughout the lifespan of the processor boards **300** to control which parameters and which revision of firmware resides on the processor boards **200.** This processor board lifespan includes the time from manufacturing and functional testing to the time at a customer facility, such as an OEM manufacturer, and finally to the time "in the field" at an end-user facility, such as a hospital or medical center.

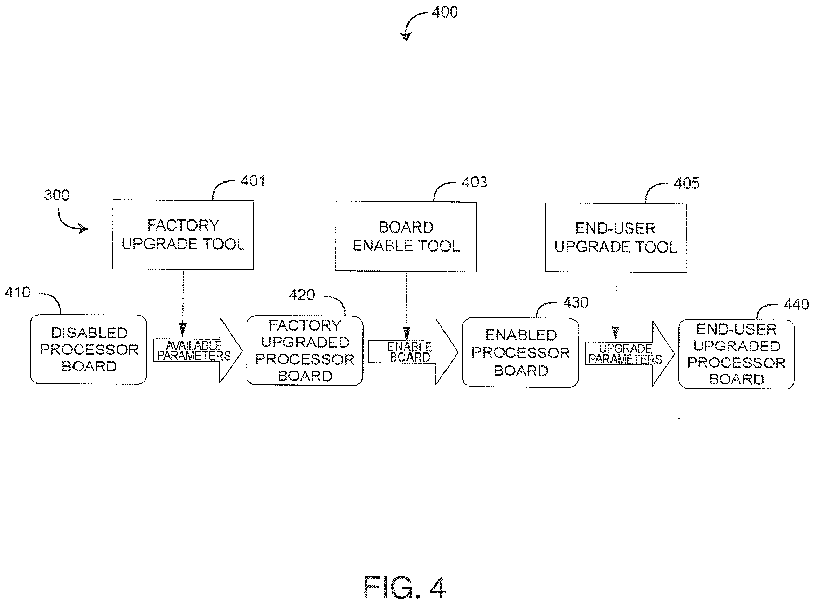

As shown in **FIG. 1****,** upgrade tools **300** are hardware devices that provide a functional interface to processor boards **200.** Upgrade tools **300** include a factory upgrade tool, a board enable tool, an end-user upgrade tool and a demo tool, as described with respect to **FIG. 4****,** below. Each upgrade tool **300** operates independently from other tools and from the application programs **100** to perform a unique function or set of functions according to its firmware configuration. These functions include processor board firmware updates, parameter upgrades and the final enabling of processor boards. Upgrade tools **300** also retrieve logged data from the processor boards **200** they have upgraded.

Also shown in **FIG. 1****,** a manufacturer application **110,** configured to run on a PC for example, is used to initially configure the upgrade tool's functionality and assign the appropriate type and number of allowed parameter upgrades and firmware version updates. The manufacturer application **110** also reads and reports on the current status of an upgrade tool **300.** Further, the manufacturer application **110** collects and documents logged data from the upgrade tool itself as well as logged data that the upgrade tool collected from processor boards **200** during upgrade sessions.

Further shown in **FIG. 1****,** a field application **120,** also configured to run on a PC, is used to read from and report on the current status of an upgrade tool **300** in the field, i.e. at an end-user facility. The field application **120** performs reading, collecting and reporting operations similar to the manufacturer application **110.** The field application **120** also sends collected data and status back to the manufacturer, such as by email or other Internet connection. In an embodiment, the field application **120** is not be capable of configuring an upgrade tool **300.**

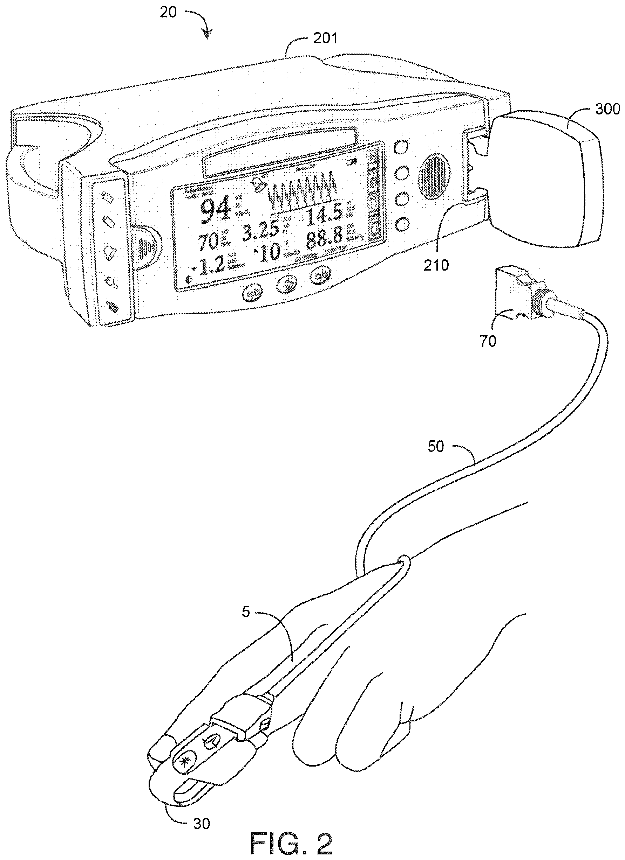

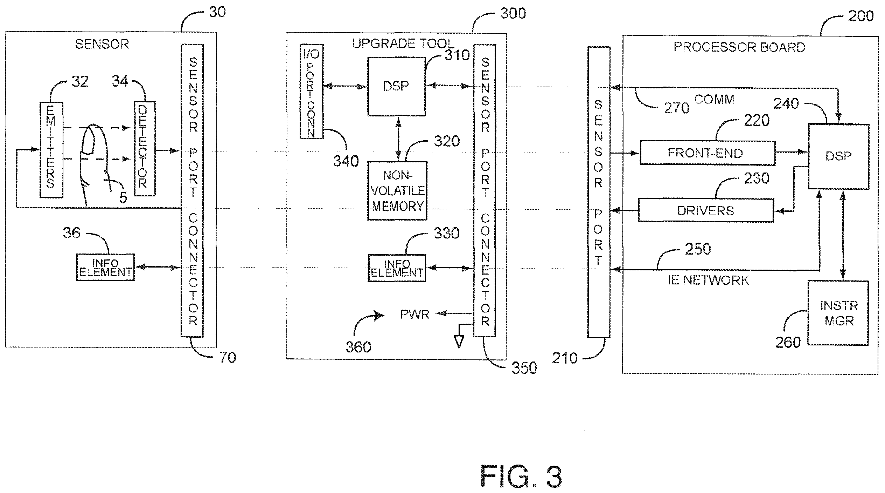

**FIG. 2** illustrates a physiological monitoring system **20** including a physiological monitor **201,** a sensor **30** and an interconnecting cable **50** having a monitor connector **70.** The sensor **30** attaches to a patient tissue site **5,** such as a fingertip. In an operational configuration (not shown), the monitor connector **70** connects to a sensor port **210** on the monitor **201.** The monitor **201** operates in conjunction with the sensor **30** so as to measure and display physiological parameters of a living being, such as a patient, as described above and in further detail below. In particular, the sensor **30** is in communications with an internal processor board **200** (**FIG. 3**) via a sensor port **210,** so that the processor board **200** (**FIG. 3**) can calculate physiological parameters responsive to sensor signals. In an upgrade configuration as shown, an upgrade tool **300** connects to the sensor port **210** on the monitor **201** so as to communicate with a processor board **200** (**FIG. 3**), upgrade processor board parameters or enable the processor board, as described in further detail below. A sensor port usable as an input/output port is disclosed in U.S. App. No. 10/898,680, filed on July 23, 2004, titled Multipurpose Sensor Port, which is assigned to Masimo Corporation and incorporated by reference herein.

**FIG. 3** illustrates a block diagram of an upgrade tool **300** incorporated with physiological monitoring system **20** (**FIG. 2**). The portion of the physiological monitoring system shown includes a sensor **30** and a processor board **200.** In an operational mode, the sensor port connector **70** of the sensor **30** connects to a monitor sensor port **210,** which is wired to the processor board **200.** In this manner, the processor board **200** communicates with the sensor **30** to receive one or more intensity signal(s) indicative of one or more physiological parameters. The processor board **200** also communicates with a host instrument (not shown) via an instrument manager **260** so as to display determined parameter values calculated using the one or more intensity signals. According to an embodiment, the processor board **200** comprises processing circuitry arranged on one or more printed circuit boards capable of installation into a physiological monitor **201** (**FIG. 2**) or capable of being distributed as some or all of one or more OEM components for a wide variety of host instruments monitoring a wide variety of patient parameters. In an embodiment, the processor board **200** comprises drivers **230,** a front-end **220,** a digital signal processor ("DSP") **240** and an instrument manager **260.** In general, the drivers **230** convert digital control signals into analog drive signals capable of driving sensor emitters **32.** The front-end **220** converts composite analog intensity signal(s) from light sensitive detector(s) **34** into digital data input to the DSP **240.** The DSP **240** has associated non-volatile memory (not shown) that stores firmware executed by the DSP, such as for deriving physiological parameter measurements.

As shown in **FIG. 3****,** the sensor **30** includes a plurality of emitters **32** irradiating a tissue site **5** with differing wavelengths of light, and one or more detectors **34** capable of detecting the light after attenuation by the tissue **5.** The processor board **200** inputs a corresponding sensor signal and is configured to determine the relative concentrations of blood constituents such as HbO₂, Hb, HbCO, HbMet and derive parameters such as fractional oxygen saturation, Hbt and blood glucose to name a few. For example, the sensor may be as described in U.S. App. No. 11,367,013 titled *Multiple Wavelength Sensor Emitters,* cited above.

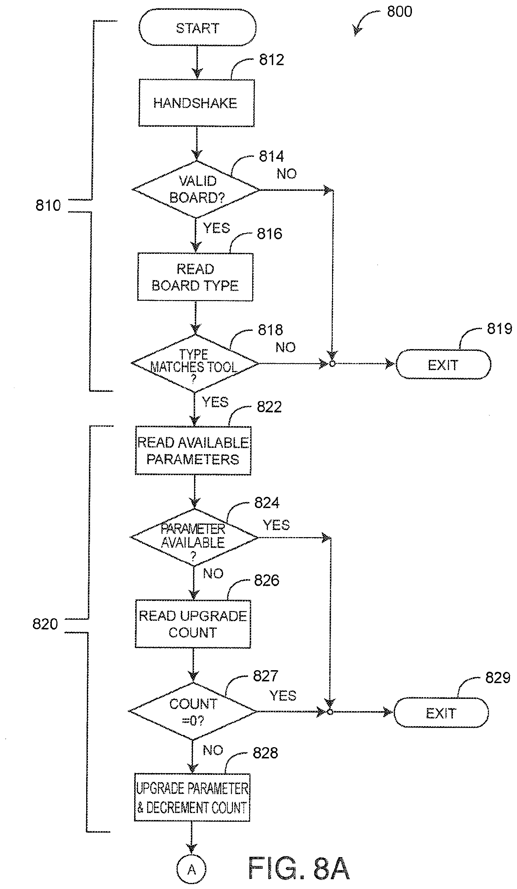

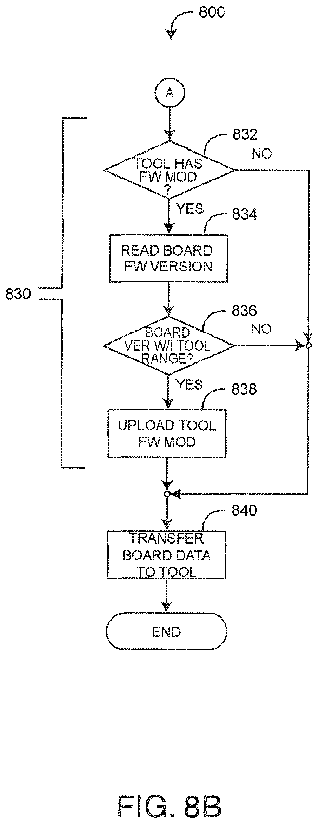

**FIG. 3** also illustrates an upgrade tool **300,** which can be programmed as a factory upgrade tool **401** (**FIG. 3**), a board enable tool **403** (**FIG. 3**), or an end-user upgrade tool **405** (**FIG. 3**). The upgrade tool **300** has a DSP **310,** nonvolatile memory **320,** an information element **330,** an I/O port connector **340** and a sensor port connector **350.** The DSP **310** performs the various upgrade tool functions, described with respect to **FIGS. 8-10****,** below. The nonvolatile memory **320** stores upload and download data transmitted to and received from the I/O port **340** via an external communications path. The nonvolatile memory **320** also stores upload and download data transmitted to and received from the sensor port connector **350** via the COMM communications path **270.** In an embodiment, an information element **330** may be, for example, a Dallas Semiconductor DS2506 EPROM available from Maxim integrated Products, Inc., Sunnyvale, CA, or equivalent. In an embodiment, IE NETWORK **\250** comprises a signal conductor for transmitting and receiving serial data and a corresponding ground conductor. An information element network is described in U.S. Pat. App. No. 11/367,036, filed March 1, 2006 entitled Configurable Physiological Measurement System, which is assigned to Masimo and incorporated by reference herein. In an embodiment, the DSP is a SHARC processing device, such as available from Analog Devices. In an embodiment, COMM **270** is a bidirectional synchronous serial communications path such as implemented by one or more SPORTs (synchronous serial ports) on a SHARC DSP. In an embodiment, the I/O port connector **340** mechanically conforms with and the signals communicated thereby electrically conform with the USB (Universal Serial Bus) standard. In embodiments, the I/O port connector **340** may mechanically conform, and the signals communicated thereby electrically conform, with any of many other serial or parallel, wired or wireless interfaces, such as RS-232, IEEE-488, SCSI, IEEE 1394 (Firewire), IEEE 802.11 and expansions thereof and IEEE 802.15 (Bluetooth), to name just a few.

In an embodiment, the I/O port connector **340** mechanically conforms, and the signals communicated thereby electrically conform, with the Ethernet network standard (IEEE 802.3). Although the update tool is shown with a single I/O port connector **340,** the update tool **300** may have multiple I/O ports conforming to various interface and network standards. Further, the update tool **300** may have one or more wireless transceivers in communications with the DSP **310** that conform to one or more wireless standards, such as IEEE 802.11 and expansions thereof and Bluetooth.

Further shown in **FIG. 3****,** in addition to adding parameters to the processor board, the upgrade tool **300** can be used to update processor board firmware and download processor board data. In particular, the upgrade tool **300** communicates via a sensor port connector **350** with a physiological monitor **201** (**FIG. 2**) and via an I/O port connector **340** with a digital I/O device. The digital I/O device may be a PC, PDA, cellphone, pager, computer-on-wheels (COW) to name a few, or other device having a data memory and interface for communicating with the upgrade tool **300.** In an embodiment, the upgrade tool **300** downloads firmware updates from a digital I/O device to nonvolatile memory **320** and uploads those updates to a physiological monitor **210** (**FIG. 2**). In an embodiment, the upgrade tool **300** downloads processor board data to nonvolatile memory **320** and uploads that data to a digital I/O device.

Also shown in **FIG. 3****,** the processor board **200** reads the info element **330** to identify the upgrade tool as such. Once identified, the processor board **200** provides power **360** to the tool. The tool DSP **310** then communicates with the board DSP **240** so as to identify the type of upgrade tool, as described with respect to **FIG. 4****,** below.

In an embodiment, processor board data includes measurement data, operational information or manufacturer information, which can be advantageously uploaded to a PC or other digital I/O device connected to the upgrade tool **300,** as described above. Measurement data may comprise patient data including raw sensor data and trend data for any one or more of the measured parameters. Operational information may comprise, for example, dates and times of operation, total operating time, failure codes and event information. Failure codes indicate, for example, processor board failures and host instrument failures. Event information includes alarm data, such as a probe off occurrence and parameter measurements outside of preset limits. Manufacturer information may comprise, as examples, service information, firmware version updates and parameter upgrade dates. Service information may include firmware upgrade history and service history, including dates and times. Processor board data may also comprise processor board identification, operational information, service information and measurement data. Board identification may include serial number and current firmware version. In an embodiment, an upgrade tool may require a significant deposit so as to encourage return to the OEM for downloading the tool data and for reuse.

In various embodiments, the upgrade tool may be connected to both a digital I/O device and a physiological monitor; the upgrade tool may be connected first to one or more digital I/O devices and then to one or more physiological monitors; or the upgrade tool may be connected to one or more physiological monitors and then to one or more digital I/O devices.

### Demo Tool

A demo tool (not shown) embodiment has only an information element **330** and a sensor port connector **350.** The information element **330** identifies the demo tool to the processor board **200** via the IE NETWORK **250.** A processor board **200** reading the information element **330** of a connected demo tool outputs simulated measurements for available parameters. This is particularly advantageous for a disabled processor board **410-420** (**FIG. 4**), i.e. a board unable to output measurements for available parameters until the board is enabled. In this manner, a customer or other user can verify that all desired parameters are available before creating an enabled board **430** (**FIG. 4**) with a board enable tool **403** (**FIG. 4**), which locks-out further parameter upgrades with a factory upgrade tool **401** (**FIG. 4**), as described below.

### Upgrade Process

**FIG. 4** illustrates a parameter upgrade process **400** where a processor board **200** (**FIG. 3**) undergoes multiple upgrade stages, including a disabled board **410,** a factory upgraded board **420,** an enabled board **430** and an end-user upgraded board **440.** Multiple upgrade tools **300** are used to transition processor boards **200** (**FIG. 3**) between stages, including a disabled processor board **410,** a factory upgraded processor board **420,** an enabled processor board **430** and an end-user upgraded processor board **440,** as described below. The upgrade tools **300** include a factory upgrade tool **401,** a board enable tool **403** and an end-user upgrade tool **405.**

In an embodiment, to facilitate the control of parameters, the processor board firmware associates a state with each parameter. Each parameter state will track whether the parameter is "available" or not. The processor board firmware will also associate a state to the entire board. This state will track whether the entire board is "enabled" or not. Disabled boards will not output measurement data for any parameter. Once enabled, boards will output measurement data for available parameters only. Additional parameters can then be upgraded to the available state. The lifespan of a processor board can be broken into four upgrade phases with regard to an example use of the parameter upgrade system, described below.

### Disabled Processor Board

A disabled processor board **410** is a newly manufactured processor boards that has passed a function test and has been programmed with released firmware. The released firmware is capable of measuring all parameters but in this initial state, a disabled processor board **410** has no "available" parameters and is "disabled," which means that no parameter data is output from the board. In an embodiment, a functional test may upgrade a disabled processor board **410** to have a default set of available parameters, such as oxygen saturation, pulse rate and perfusion index. In another embodiment, a disabled processor board **410** is shipped to a customer with no parameters available, and the customer adds the default parameters along with additionally purchased parameters using one or more factory upgrade tools **401,** as described below.

### Factory Upgraded Processor Board

An upgrade tool that has been configured to function as a factory upgrade tool **401** can be used to upgrade the disabled processor board **410** to a factory upgraded processor board **420.** This upgrade is with a single parameter, such as HbCO for example. After this upgrade, HbCO is referred to as being available. The board itself is still disabled and will not output HbCO parameter data. During this phase, multiple parameters can be upgraded to be available. In an embodiment, a different factory upgrade tool **401** must be used for each parameter. Available parameters for unenabled boards **410, 420** can be verified in a demo mode using a demo tool as described above.

### Enabled Processor Board

An upgrade tool that has been configured to function as a board enable tool **403** is used to "enable" the factory upgraded processor board **420** to an enabled processor board **430.** An enabled board **330** is capable of sending parameters to a host instrument. After the board is enabled, any available parameters will be measured and output with the appropriate sensors. Once a board is enabled, it can no longer be upgraded with a factory upgrade tool **401.** From this point on, only an end-user upgrade tool **405** can be used to upgrade the board with additional parameters or firmware updates.

### End-User Upgraded Board

This phase represents the rest of a processor board's lifespan. Additional parameters can be added to an enabled processor board **430** with an end-user upgrade tool **405** only and is designated an end-user upgraded processor board **440.** In an embodiment, as with the factory upgrade tool **401,** a different end-user upgrade tool **405** must be used for each parameter to be added. All available parameters will remain available for the remaining lifespan of the board. In an embodiment, an upgrade tool **400** also can be used during the upgrade processes described above to advantageously update processor board firmware to the latest version and to retrieve various processor board data from one or more processor boards, as described in detail below. The above describes but one example use of the parameter upgrade system.

### Upgrade Tool Configuration

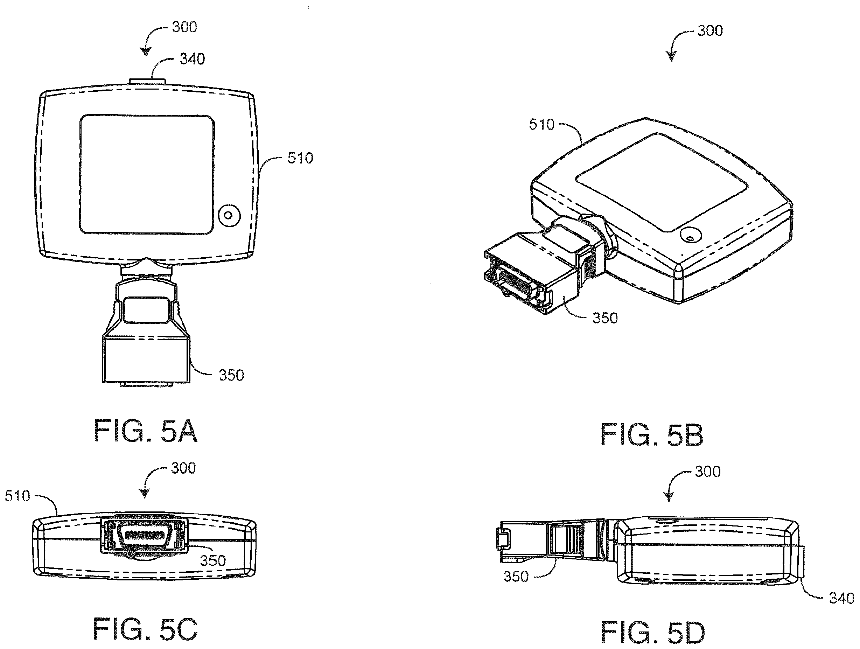

**FIGS. 5-7** illustrate an upgrade tool **300** embodiment. As shown in FIGS. 5A-D, an upgrade tool **300** has a case **510,** an I/O port connector **340** and a sensor port connector **350,** as described above. In the embodiment shown, the I/O port connector **340** is a USB connector, and the sensor port connector **350** is a 20-pin connector.

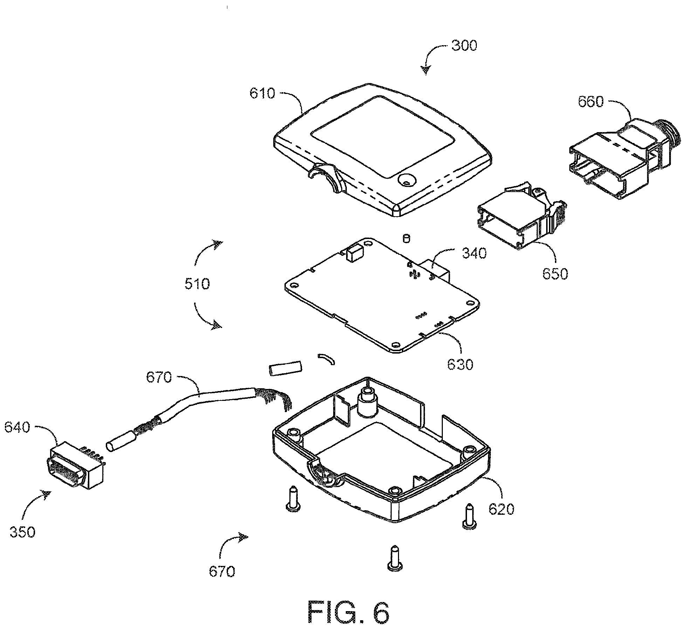

As shown in **FIG. 6****,** the case **510** has an upper cover **610** and a lower cover **620,** which are attached together with fasteners **670** to enclose a circuit board **630.** The circuit board **630** mechanically mounts and electrically connects the DSP **310** (**FIG. 3**), non-volatile memory **320** (**FIG. 3**) and info element **330** (**FIG. 3**) and associated "glue" circuits, conductors and components. The sensor port connector **350** has a connector block **640,** a clip **650,** a shell **660** and a cable **670.** The cable **670** interconnects the connector block **640** and the circuit board **630.** The connector block **640** provides pins for electrically attaching wires from one end of the cable **670** and connector contacts for mating with corresponding sensor port **210** (**FIG. 1**) contacts. The clip **650** provides a finger releasable hold to the sensor port **210** (**FIG**. **1**) connector. The shell **660** houses the connector block **640** and clip **650** and provides a strain relief mount to the case **510.**

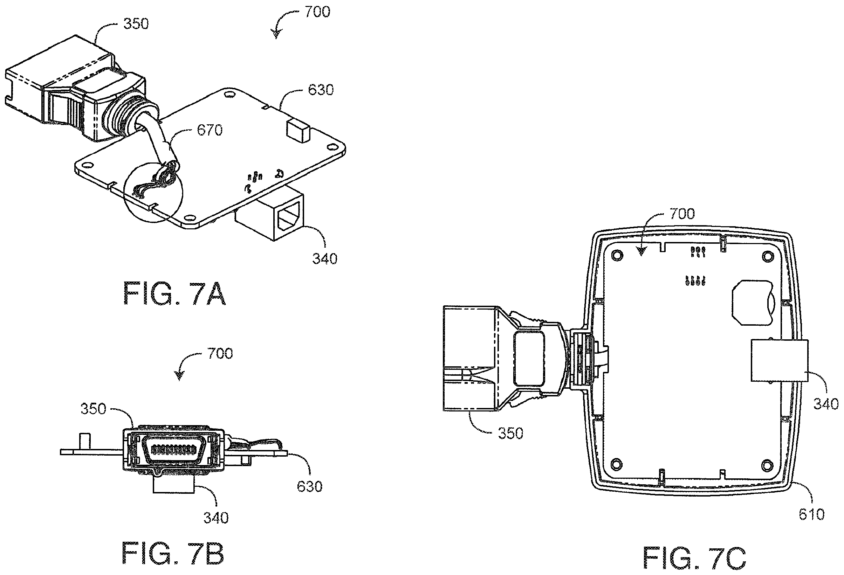

As shown in FIGS. 7A-C, a circuit board assembly **700** has the sensor port connector **350** mounted to the circuit board **630** via the cable **670.** The circuit board assembly **700** mounts into the upper cover **610** so that the circuit board **630** is enclosed within the case **510** (**FIG. 6**) and secured by the fasteners **670** (**FIG. 6**) and so that the I/O port connector **340** and sensor port connector **350** are exposed.

### Upgrade Tool Functions

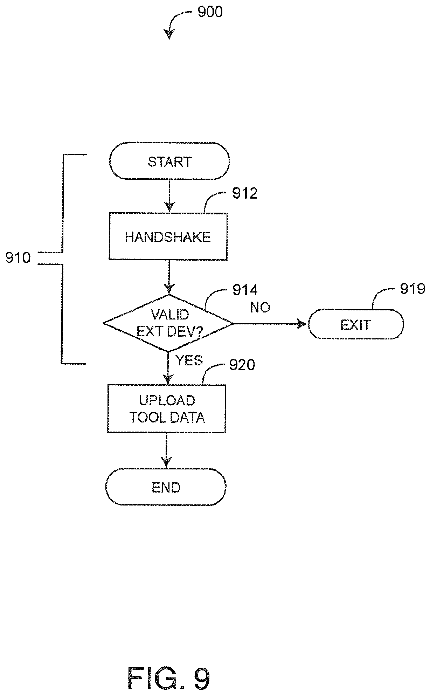

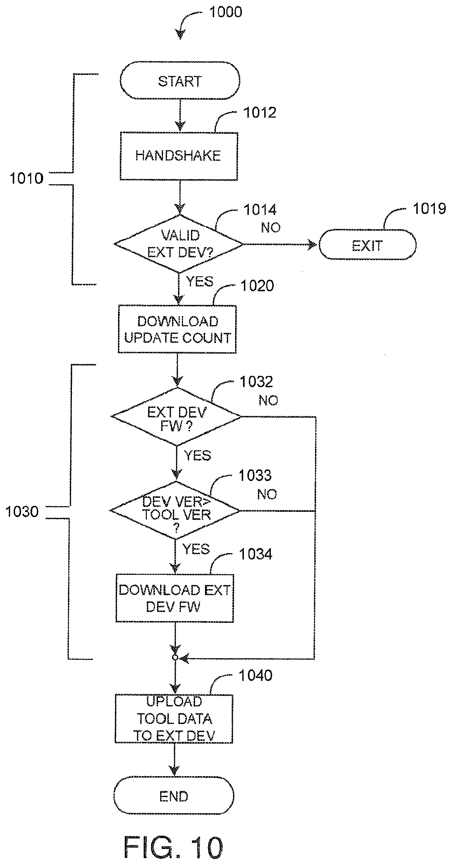

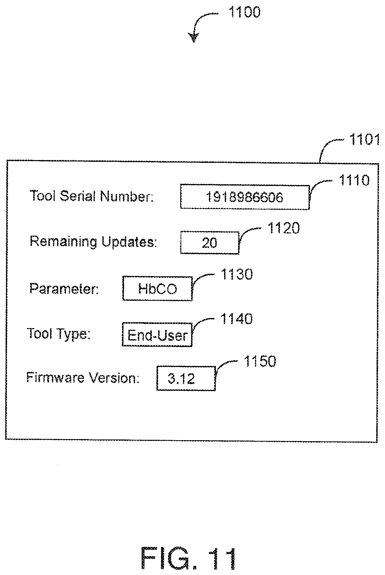

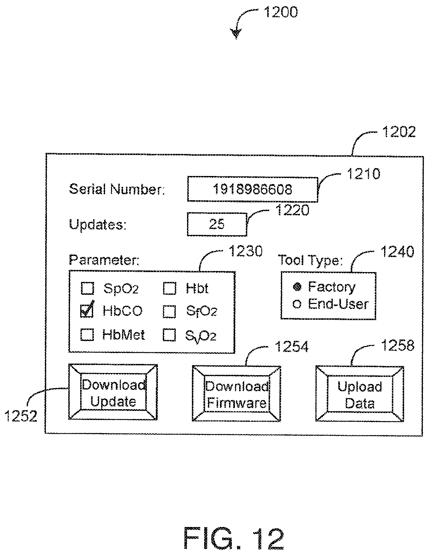

**FIGS. 8-10** illustrate upgrade tool functions, which include processor board functions 800 (FIGS. 8A-B), tool reading functions **900** (**FIG. 9**) and tool maintenance functions **1000** (**FIG. 10**). Corresponding graphical user interfaces (GUIs) are described with respect to **FIGS. 11** and **12****,** below. As shown in FIGS. 8A-B, processor board functions **800** involve upgrade tool **300** (**FIG. 3**) communications with a processor board **200** (**FIG. 3**) via the COMM path **270** (**FIG. 3**) and the sensor port **210** (**FIG. 3**). Processor board functions **800** include processor board authentication **810,** parameter upgrade administration **820,** uploading processor board firmware modifications **830** and downloading processor board data **840.** Processor board authentication **810** includes verification that an valid processor board is connected to the sensor port. This verification includes transmission and receipt of an encrypted handshake **812** between the upgrade tool and the processor board. This handshake is successful only if the upgrade tool recognizes the processor board **814** and the processor board recognizes the tool. Processor board authentication **810** also includes board type determination **816** and matching **818** the upgrade tool type, i.e. factory tool or end-user tool to the processor board type, i.e. a disabled board **410, 420** (**FIG. 4**) or an enabled board **430, 440** (**FIG. 4**). If the processor board does not authenticate or there is a mismatch between tool type and board type, e.g. a factory tool is connected to an enabled board, then the upgrade tool performs no action **819** with respect to the connected processor board.

As shown in FIGS. 8A-B, parameter upgrade administration **820** includes reading the available parameters **822** from the processor board and verifying the tool upgrade count **826.** If and only if the tool specific parameter is currently unavailable on the processor board **824** and the tool upgrade count is not zero **827,** is the processor board parameter made available. Otherwise, the upgrade tool performs no action **829.** The upgrade count is decremented accordingly **828.**

Further shown in FIGS. 8A-B, uploading processor board firmware modification **830** includes determining if the tool contains a firmware modification **832,** reading the board firmware version number **834** and replacing the processor board firmware with the tool firmware modification **838** if and only if the processor board version number is within the range defined in the tool **836.** The downloading processor board data **840** includes transferring the processor board data into the tool and storing the data in tool nonvolatile memory according to a processor board identifier.

**FIG. 9** illustrates the tool reading functions **900,** which involve communications with an external digital device, such as a PC, via the I/O port **340** (**FIG. 3**). External device authentication **910** verifies that an authorized external device is accessing an upgrade tool. This verification includes receipt and transmission of an encrypted handshake between the upgrade tool and the external device **912.** This handshake is successful only if the upgrade tool recognizes the external device **914.** Otherwise, the tool is non-responsive **919** to the attached device. The upload tool data **920** transfers tool specific data to the external device, such as described with respect to **FIG. 10****,** below.

**FIG. 10** illustrates the tool maintenance functions **1000,** which also involve communications with an external digital device, such as a PC, via the I/O port **340** (**FIG. 3**). Tool maintenance functions **1000** include external device authentication **1010,** downloading parameter upgrades **1020** and firmware modifications **1030** from the external digital device and uploading processor board history **1040** to the external digital device. External device authentication **1010** verifies that an authorized external device is accessing an upgrade tool. This verification includes receipt and transmission of an encrypted handshake between the upgrade tool and the external device **1012.** This handshake is successful only if the upgrade tool recognizes the external device **1014.** Otherwise, the tool is non-responsive **1019** to the attached device. The parameter upgrade download **1020** indicates the tool parameter and the number of authorized parameter upgrades for that parameter. Further, if the external device has a processor board firmware update **1032,** that firmware is downloaded into the tool **1034.** Also, any processor board data previously downloaded into the tool from one or more processor boards is uploaded into the external device **1040.**

### PC Interface

**FIGS. 11-12** illustrate a graphical user interfaces (GUIs) for a field application **120** (**FIG. 1**) and a manufacturer application **110** (**FIG. 1**), respectively. As shown in **FIG. 11****,** in a field application GUI **1100** embodiment, a PC provides an interface for a customer or end-user to "read" a factory upgrade tool **401** (**FIG. 4**) or an end-user tool **405** (**FIG. 4**). In particular, a user can determine a tool serial number **1110,** the remaining number of upgrades **1120,** the tool parameter **1130,** the tool type **1140** and the firmware version **1150.** The process is read only, i.e. the user cannot alter the tool or read other data stored in the tool.

As shown in **FIG. 12****,** in a manufacturer GUI **1202** embodiment, a PC provides an interface for a manufacturer to both read and modify a tool. The tool serial number **1210** can be displayed. The number of upgrades **1220,** the tool parameter **1230** and the tool type **1240** can also be displayed and modified.

### Networking and Wireless Applications

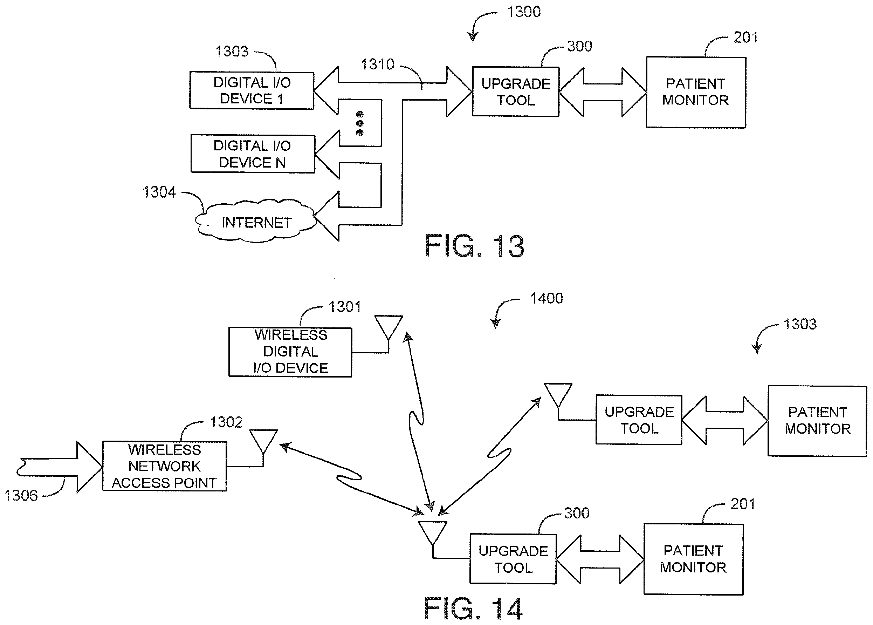

**FIG. 13** illustrates an upgrade tool networking application **1300.** An upgrade tool **300** interconnects a physiological monitor **201** via a sensor port **210** (**FIG. 3**) with a network **1310** via an I/O port **340** (**FIG. 3**), such as an Ethernet compatible interface. In this manner, the upgrade tool **300** can communicate with one or more digital I/O devices **1303,** as described above, or gain access to the Internet **1304.** In an embodiment, when the upgrade tool **300** is connected to the physiological monitor **201,** the upgrade tool accesses a central website via the network **1310** (or a wireless connection as described below) and the Internet **1304** so as to download the latest firmware updates, which are made accessible from the website. These firmware updates are then uploaded to a corresponding processor board within the physiological monitor **201,** as described above.

**FIG. 14** illustrates an upgrade tool networking application **1400.** In an embodiment, an upgrade tool **300** interconnects a physiological monitor **201** via a sensor port **210** (**FIG. 3**) with a wireless transceiver via an I/O port **340** (**FIG. 3**). The wireless transceiver is compliant with a wireless standard, such as IEEE-802.11 or IEEE 802.15 (Bluetooth). In an embodiment, the upgrade tool **300** provides wireless communications with a wireless digital I/O device **1301.** In an embodiment, the upgrade tool **300** provides wireless communications with a wireless network access point **1302.** In an embodiment, the upgrade tool **300** also has a network I/O port in communications with a network, such as described with respect to **FIG. 13****,** above, and acts as a network access point for a second wireless upgrade tool connected to a second physiological monitor **1303.** In other embodiments, an upgrade tool connected to any power source and a wired or wireless downloads firmware updates or any other data and uploads stored data while in communication with a manufacturer server or other secure computer.

### Tiered Parameter Pricing

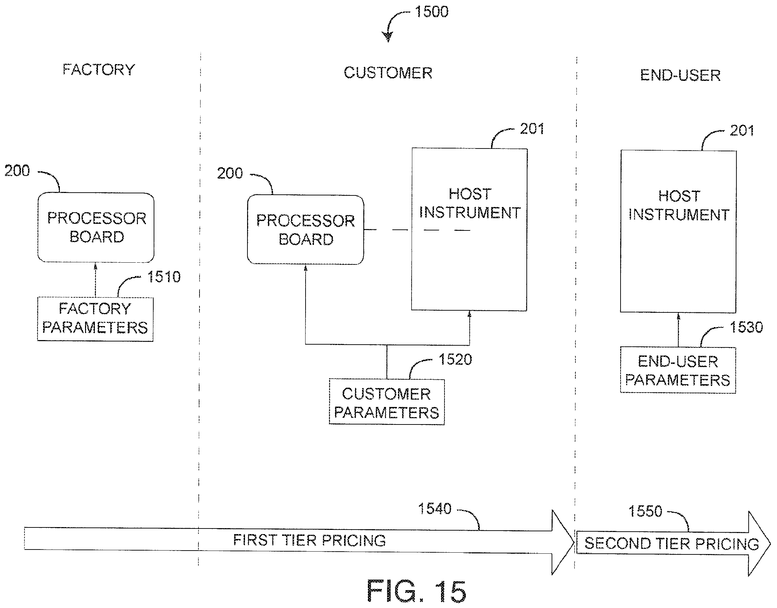

**FIG. 15** illustrates two-tiered parameter pricing for a processor board **200,** such as described with respect to **FIG. 3****,** above. As described above, the processor board **200** has the capability to measure multiple physiological parameters. As described above, a parameter upgrade process **400** (**FIG. 4**) provides a flexible pricing plan for these multiple parameters. In an embodiment, parameters can be made available individually to individual boards, providing processor boards that are custom-configurable to fit customer needs.

In an embodiment, parameter programming can occur at a factory, a customer or an end-user facility. Factory parameters **1510** include default parameters added to a newly manufactured processor board **200.** Customer parameters **1520** include additional parameters added to a processor board **200** in conjunction with the incorporation of the processor board within a host instrument **201,** as described with respect to **FIGS. 3-4****,** above. End-user parameters **1530** include additional parameters that are made available to an enabled processor board **200** integrated into an operational host instrument **201** sold or otherwise provided to an end-user, such as a hospital or medical facility.

In an advantageous embodiment, a parameter upgrade system is configured so as to provide a self-enforcing, two-tier parameter pricing structure. A first tier pricing **1540** applies to factory parameters **1510** and customer parameters **1520.** A second tier pricing **1550** applies to end-user parameters **1530.** As one example, first tier pricing **1540** applies a lower price for one or more of the available parameters as compared to the second tier pricing **1550.**

A parameter upgrade system has been disclosed in detail in connection with various embodiments. These embodiments are disclosed by way of examples only and are not to limit the scope of the claims that follow. One of ordinary skill in art will appreciate many variations and modifications. For example, in an embodiment, upgrade tools **300** (**FIG. 3**) spread firmware updates between processor boards in a viral manner, i.e. downloading a detected higher version firmware from a processor board and uploading the firmware to other processor boards that have lower version firmware.

## Claims

1. A parameter upgrade system comprising:
a physiological monitor (201) having a sensor port (210) configured to attach and communicate with a sensor (30);
a processor board (200) in communications with the sensor port having a board digital signal processor (DSP) (240);
a plurality of firmware instructions residing on the processor board and executable by the board DSP so as to calculate a plurality of physiological parameters in response to a sensor signal received from the sensor and determine an enable state for each of the plurality of physiological parameters; and
a plurality of handheld upgrade tools (401, 403, 405) individually attachable to the sensor port in lieu of the sensor, wherein each of the plurality of handheld upgrade tools comprises:
a tool digital signal processor (DSP) (310) configured to provide changes to the plurality of firmware instructions and configured to, update, based on a verification that a parameter upgrade is available, an enable state of a physiological parameter to make the physiological parameter available for output by the monitor, wherein making the physiological parameter available for output by the monitor enables the sensor port to calculate at least one additional physiological parameter in response to the sensor;
a sensor port connector (350) that mates directly with the sensor port and provides a communications path between the tool DSP and the board DSP;
an I/O port connector (340) that provides communications between the tool DSP and an external digital device; and
an information element (330) that identifies the handheld upgrade tool to the board DSP.

2. The parameter upgrade system according to claim 1, wherein the parameter upgrade is available when an enable state of the physiological parameter is currently unenabled on the processor board and wherein a tool upgrade count is nonzero.

3. The parameter upgrade system according to claim 1 wherein at least one of the handheld upgrade tools comprise a factory upgrade tool having the tool DSP programmed to communicate to the board DSP to make a specific physiological parameter available to the physiological monitor.

4. The parameter upgrade system according to claim 3 wherein at least one of the handheld upgrade tools comprise a board enable tool having the tool DSP programmed to enable the board DSP to calculate available physiological parameters to be displayed by the physiological monitor.

5. The parameter upgrade system according to claim 4 wherein at least one of the handheld upgrade tools comprise an end-user tool having the tool DSP programmed to communicate to the board DSP to add available physiological parameters after the board DSP has been enabled.

6. The parameter upgrade system according to claim 5 wherein at least one of the handheld upgrade tools comprise a demo tool to indicate to the board DSP to verify available parameters.

7. The parameter upgrade system according to claim 6 further comprising a manufacturer application executing on the PC and in communications with the tool DSP via the I/O port connector, wherein the manufacturer application programs the tool DSP with at least one of a tool parameter and a number of parameter updates that can be made with the tool.

8. The parameter upgrade system according to claim 7 further comprising a field application executing on a PC and in communications with the tool DSP via the I/O port connector, wherein the field application displays on the PC at least one of the tool parameter and the number of parameter updates that can be made with the tool.
